(19) **Europäisches Patentamt**
**European Patent Office**
**Office européen des brevets**

(11) **EP 2 405 808 B1**

(12) **EUROPÄISCHE PATENTSCHRIFT**

(45) Veröffentlichungstag und Bekanntmachung des Hinweises auf die Patenterteilung:
**02.10.2019 Patentblatt 2019/40**

(21) Anmeldenummer: **10701815.2**

(22) Anmeldetag: **25.01.2010**

(51) Int Cl.:
**A61B 5/0402** (2006.01)

(86) Internationale Anmeldenummer:
**PCT/EP2010/000417**

(87) Internationale Veröffentlichungsnummer:
**WO 2010/102695 (16.09.2010 Gazette 2010/37)**

(54) **VERFAHREN UND VORRICHTUNG ZUM AUFNEHMEN EINES ELEKTROKARDIOGRAMMS**

METHOD AND DEVICE FOR RECORDING AN ELECTROCARDIOGRAM

PROCÉDÉ ET DISPOSITIF PERMETTANT L'ÉTABLISSEMENT D'UN ÉLECTROCARDIOGRAMME

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **09.03.2009 DE 102009012352**

(43) Veröffentlichungstag der Anmeldung:
**18.01.2012 Patentblatt 2012/03**

(73) Patentinhaber: **Personal Medsystems GmbH 10709 Berlin (DE)**

(72) Erfinder:
• **RIEMENSCHNEIDER, Markus 65232 Taunusstein (DE)**
• **BONAVENTURA, Klaus 14129 Berlin (DE)**
• **BRAND, Felix 60322 Frankfurt am Main (DE)**

(74) Vertreter: **Samson & Partner Patentanwälte mbB Widenmayerstraße 6 80538 München (DE)**

(56) Entgegenhaltungen:
**EP-A1- 0 712 605    US-A1- 2007 232 946**

• **KORS J ET AL: "Accurate Automatic Detection of Electrode Interchange in the Electrocardiogram" AMERICAN JOURNAL OF CARDIOLOGY, Bd. 88, Nr. 4, 15. August 2001 (2001-08-15), Seiten 396-399, XP002574018 ISSN: 0002-9149**
• **BALÁZS G ET AL: "Intelligent cardiac telemonitoring system" COMPUTERS IN CARDIOLOGY, 2004 CHICAGO, IL, USA SEPT. 19-22, 2004, PISCATAWAY, NJ, USA,IEEE, 19. September 2004 (2004-09-19), Seiten 745-748, XP010814133 ISBN: 978-0-7803-8927-4**

EP 2 405 808 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

GEBIET DER ERFINDUNG

Die vorliegende Erfindung bezieht sich allgemein auf ein Verfahren und eine Vorrichtung zur Messung von elektrischen Herzsignalen und insbesondere auf ein Verfahren und eine Vorrichtung zum Aufnehmen eines Elektrokardiogramms.

HINTERGRUND DER ERFINDUNG

Es sind allgemeine Vorrichtungen und Verfahren zum Messen der elektrischen Aktivitäten des Herzens von Lebewesen, insbesondere des Menschen, bekannt.

Zur Messung der Herzaktivität sind sogenannten Elektrokardiographen bekannt, die mittels einzelner am zu untersuchenden Subjekt angebrachter Elektroden, elektrische Potentiale im Herzmuskel an der Hautoberfläche messen.

Die Elektroden können in verschiedenen Konfigurationen an Körperstellen angelegt bzw. platziert werden, um dort jeweils die Potentiale zu messen, d.h. abzuleiten. Es gibt verschiedene Ableitsysteme, die nicht nur eine unterschiedliche Anzahl von Elektroden verwenden, sondern auch unterschiedliche Positionen am Körper zur Ableitung der einzelnen Potentiale. Die Potentiale werden im Allgemeinen zwischen zwei vorgegebenen Elektroden gemessen. Der gemessene zeitliche Potentialverlauf bildet das sogenannte Elektrokardiogramm.

Grundsätzlich können ein Arzt oder ein automatischer Auswertealgorithmus Elektrokardiogramme nur dann sinnvoll interpretieren, wenn sie die Anordnung der Elektroden kennen und wissen, zwischen welchen Elektroden die einzelnen Potentiale gemessen wurden.

Wie beispielhaft der Stand der Technik DE 100 65 578 A1 zeigt, passiert es in der Praxis oftmals, dass die Elektroden nicht genau in der für das Ableitsystem vorgegebenen Position angelegt werden und dass zusätzlich die Elektroden auch vertauscht werden. Das dort offenbarte Verfahren verwendet ein Ableitsystem, das mit 10 Elektroden arbeitet, und ist in der Lage eine Vertauschung oder falsche Positionierung der Elektroden mittels einer Vektoranalyse bzw. Vektorkardiographie zu erkennen. Diese Vektoranalyse basiert auf der Zuordnung eines Vektors zu jeder Elektrode und einem Vergleich des Ergebnisses der Vektoranalyse mit einem Referenzergebnis. Bei entsprechender Abweichung erkennt das System eine Vertauschung von Elektroden.

Weiterhin ist es bekannt, bspw. mittels der Vektorkardiographie, verschiedene Ableitsysteme ineinander umzurechnen. So offenbart bspw. das US-Patent 4,850,370 ein Ableitsystem, das lediglich 4 Elektroden verwendet, in ein 10- oder 12-Kanal Elektrokardiogramm umzurechnen.

Wie bereits erwähnt, ist die Platzierung der Elektroden und vor allem, die richtige Zuordnung, d.h. die richtige "Permutation" der Elektroden, fehleranfällig. Selbst geschultes Personal wählt oftmals die falsche Permutation der Elektroden, selbst wenn diese und zugehörige Signaleingänge eines Elektrokardiographen farblich markiert sind.

Durch die Zunahme der Herzkreislauferkrankungen ist oftmals neben der stationären oder ambulanten Überwachung von Patienten auch eine Langzeit-EKG Überwachung erforderlich, bei denen die Patienten ein tragbares EKG-Gerät mit sich tragen. Ferner ist es für herzkranke Patienten sinnvoll, unabhängig von vorhandenem Fachpersonal eine EKG-Messung im Bedarfsfall durchzuführen, bspw. beim sogenannten Home Monitoring. Die Gefahr, dass der nicht fachkundige Anwender die Elektroden beim Selbstanlegen - insbesondere in Stresssituationen - vertauscht, ist deutlich höher als bei dem Fachpersonal. Gerade der nicht fachkundige Anwender bräuchte allerdings ein System, das im Bedarfs- bzw. Notfall zuverlässig ein EKG erstellt und zuverlässig ein Ergebnis ausgibt, wie bspw. eine Warnung, dass er umgehend einen Notarzt verständigen soll.

Ein anderer bekannter Weg, Permutationen der Elektroden zu vermeiden, ist die Elektrodenanordnung fest vorzugeben, indem sie bspw. durch eine Weste, einen Gürtel, ein Geschirr oder ein anderes mechanisches Mittel fix ist. Solche Lösungen sind allerdings oftmals unflexibel, da sie sich nicht einfach an verschiedene Körpergrößen und unterschiedliche anatomische Gegebenheiten anpassen lassen. Ferner sind solche System oftmals unbequem zu tragen.

Aus G. Balázs et al., "Intelligent Cardiac Telemonitoring System", Computers in Cardiology 2004; 31, S. 745-748 ist ein Verfahren bekannt, bei dem versucht wird, vertauschte Elektroden festzustellen. Dabei überprüft ein Algorithmus alle möglichen Vertauschungsvarianten und berechnet eine Korrelation mit einer gespeicherten Referenzmessung. Für die Entscheidung, ab welchem Korrelationsgrad eine Übereinstimmung zwischen der untersuchten Vertauschungsvariante und der Referenzmessung bestimmt wird, ist eine Vielzahl von personalisierten Messungen erforderlich.

Aus der US-Patentanmeldung US 2007/0232946 A1 ist ein Verfahren bekannt, bei dem eine Ableitung in eine andere transformiert wird, wobei diese Transformation für jede mögliche Vertauschung von Elektroden vorgenommen wird. Der Transformation liegt ein Dipol-Modell des Herzens zugrunde und es wird angenommen, dass die Transformation für eine falsche Vertauschung anders ist, als für die richtige Elektrodenanordnung.

Aus J. A. Kors, "Accurate Automatic Detection of Electrode Interchange in the Electrocardiogram", American Journal of Cardiology, Bd. 88, Nr. 4, 15. August 2001, Seiten 396-399, ist ebenfalls ein Verfahren zum Erkennen vertauschter Elektroden bekannt. Bei diesem Verfahren werden Redundanzen bei dem 12-Ableitungs-EKG verwendet, um andere Ableitungen daraus zu rekonstruieren. Dabei werden sowohl alle Vertauschungen des 12-Ableitungs-EKG berechnet als auch zugehörige Rekonstruktionen andere Ableitungen. Dann werden Korrelationen zwischen beliebigen Ableitungen und ihren Rekonstruktionen berechnet, da angenommen wird, dass die Korrelation zwischen einer Ableitung und ihrer Rekonstruktion dann am höchsten ist, wenn die richtige Elektrodenanordnung verwendet wird.

Aufgabe der vorliegenden Erfindung ist es, eine verbesserte Vorrichtung und ein verbessertes Verfahren zum Aufnehmen eines Elektrokardiogramms zur Verfügung zu stellen.

ZUSAMMENFASSUNG DER ERFINDUNG

Nach einem ersten Aspekt stellt die vorliegende Erfindung ein Verfahren zum Aufnehmen eines Elektrokardiogramms nach dem unabhängigen Anspruch 1 bereit.

Nach einem weiteren Aspekt stellt die vorliegende Erfindung eine Elektrokardiogrammvorrichtung zum Aufnehmen eines Elektrokardiogramms nach dem unabhängigen Anspruch 8 bereit.

Weitere Aspekte und Merkmale der vorliegenden Erfindung ergeben sich aus den abhängigen Ansprüchen, der beigefügten Zeichnung und der nachfolgenden Beschreibung bevorzugter Ausführungsbeispiele.

KURZBESCHREIBUNG DER ZEICHNUNG

Ausführungsbeispiele der Erfindung werden nun beispielhaft und unter Bezugnahme auf die beigefügte Zeichnung beschrieben, in der:

Fig. 1 ein Ableitungssystem mit einer ersten Elektrodenanordnung veranschaulicht;

Fig. 2 den mit der Elektrodenanordnungen nach Fig. 1 gemessenen Potentialverlauf als Referenz-EKG zeigt;

Fig. 3 das Ableitsystem nach Fig.1 mit einer zweiten Elektrodenanordnung veranschaulicht;

Fig. 4 den mit der Elektrodenanordnungen nach Fig. 3 gemessenen Potentialverlauf zeigt;

Fig. 5 einen Vergleich des Potentialverlaufes von Fig. 4 mit dem Referenz-EKG aus Fig. 2 veranschaulicht;

Fig. 6 das Ableitsystem nach Fig.1 mit einer dritten Elektrodenanordnung veranschaulicht;

Fig. 7 den mit der Elektrodenanordnungen nach Fig. 6 gemessenen Potentialverlauf zeigt;

Fig. 8 einen Vergleich des Potentialverlaufes von Fig. 7 mit dem Referenz-EKG aus Fig. 2 veranschaulicht.

Fig. 9 ein Ausführungsbeispiel einer Elektrokardiogrammvorrichtung in Übereinstimmung mit der vorliegenden Erfindung zeigt; und

Fig. 10 ein Ausführungsbeispiel eines Verfahrens zum Aufnehmen eines Elektrokardiogramms in Übereinstimmung mit der vorliegenden Erfindung veranschaulicht.

BESCHREIBUNG DER BEVORZUGTEN AUSFÜHRUNGSBEISPIELE

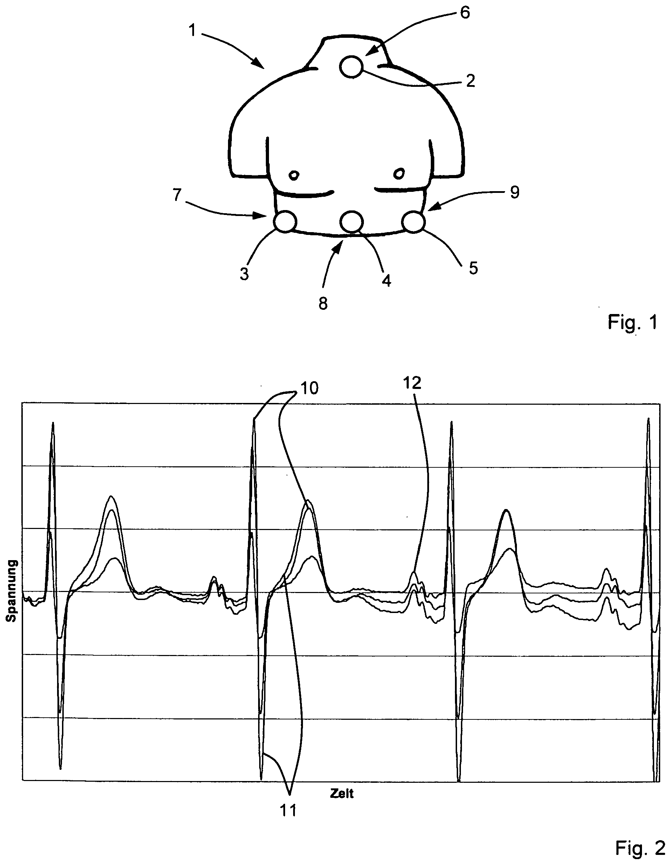

In Fig. 1 ist ein Ableitsystem 1 mit einer ersten Elektrodenanordnung von vier Elektroden 2, 3, 4, 5 veranschaulicht. Vor einer detaillierten Beschreibung folgen zunächst allgemeine Erläuterungen zu den Ausführungsbeispielen und deren Vorteile.

Wie oben bereits geschildert, kommt es in der täglichen Praxis der Anwendung von Elektrokardiographen vor, dass ein Anwender, wie Fachpersonal oder ein Patient, die Elektroden beim Anlegen vertauscht. Insbesondere für den unerfahrenen Benutzer ist die richtige Anordnung der Elektroden nicht immer leicht zu erkennen. Ferner befindet sich der unerfahrene Benutzer oftmals in einer Stresssituation, wenn er bspw. zu Hause die Elektroden eines EKG-Gerätes an sich selbst anbringt, was das Erkennen einer falschen Permutation der Elektroden weiter erschwert. Gibt dann das Gerät aufgrund der vertauschten Elektroden noch "falschen Alarm", z.B. indem es angibt, dass der Patient einen Herzinfarkt erlitten hat, kann dies den Benutzer zusätzlich - und völlig unnötig - in Panik versetzen.

Weiterhin hat der Erfinder erkannt, dass die Lösung nach der eingangs erwähnten DE 100 65 578 A1 komplexe Winkelberechnungen benötigt, um fehlerhaft positionierte Elektroden zu erkennen. Ferner ist es bei dieser Lösung notwendig, dass das Referenzergebnis zuvor bei einer fest vorgegebenen Elektrodenanordnung aufgenommen wurde, die auch nicht bezüglich der Vorgabe vertauscht sein darf. Waren die Elektroden bei Aufnahme des Referenz-Ergebnisses vertauscht, so ist es nach dieser Lösung nicht mehr möglich, eine Vertauschung der Elektroden bei einer nachfolgenden Aufnahme eines Elektrokardiogramms zuverlässig zu erkennen.

Die Ausführungsbeispiele der vorliegenden Erfindung erkennen bspw. nicht nur eine "falsche" Permutation der Elektroden, sondern sie können auch die Art der Permutation der Elektroden erkennen und sind in der Lage, bspw. durch eine Umrechnung, trotzdem das "richtige" Elektrokardiogramm (EKG) bzw. ein Abweichung des richtigen EKGs von einem Referenz-Elektrokardiogramm auszugeben. Damit ist es möglich, auch bei vertauschten Elektroden, bspw. bei der Aufnahme des aktuellen und/oder des Referenz-Elektrokardiogramms, eine physiologische Veränderung festzustellen, ohne dass ein neues EKG aufgenommen werden muss. Ferner muss bei den Ausführungsbeispielen die Anordnung der Elektroden bei Aufnahme des Referenz-Elektrokardiogramms nicht bekannt sein. Es kann also das Referenz-Elektrokardiogramm und/oder das aktuelle Elektrokardiogramm mit einer beliebigen, unbekannten Permutation der Elektroden aufgenommen werden.

Elektrokardiogramme entstehen durch Aufnehmen von elektrischen Signalen über Elektroden, welche an einem zu untersuchenden Subjekt, meist in einer vorgegebenen Position, angebracht sind. Unter Subjekt ist im Allgemeinen ein Mensch zu verstehen. Allerdings ist die vorliegende Erfindung nicht auf Menschen eingeschränkt, sondern kann bspw. auch bei Tieren angewendet werden.

Die Anzahl der Elektroden kann - je nach Ableitsystem - variieren. Verbreitet sind insbesondere Ableitsysteme, die vier, sechs und zehn Elektroden verwenden. Zur Potentialmessung benötigt man wenigstens zwei Elektroden, da letztlich immer nur ein Potentialunterschied zwischen zwei Elektroden gemessen werden kann. Das Elektrokardiogramm gibt demzufolge den zeitlichen Verlauf von Potentialunterschieden zwischen einzelnen Elektroden wieder.

Die Potentialunterschiede zwischen den Elektroden hängen unter anderem von der Positionierung der Elektroden auf oder an dem zu untersuchenden Subjekt ab, da die elektrischen Ströme, die in dem Herzmuskel fließen, sich nicht nur zeitlichen sondern auch örtlich unterscheiden.

Daher können je nach Positionierung der Elektroden und entsprechend je nach Auswertung der gemessenen Potentialunterschiede zeitlich und örtlich unterschiedliche Abläufe im Herzmuskel gemessen und beobachtet werden.

Im Folgenden sind beispielhaft einige bekannte EKG-Ableitungen erläutert, wobei die Erfindung bei jedem beliebigen Ableitsystem anwendbar ist.

Die durch die Herzströme entstehenden Potentialdifferenzen können auf unterschiedliche Weise gemessen werden. Diese Messungen werden auch als Ableitungen bezeichnet. Es ist bekannt, sie nach der Messmethodik, d.h. der Art der Ableitung und nach dem Ort, an dem die Elektroden angebracht sind, zu differenzieren:
Ableitungen kann man bspw. je nach Verschaltung der (Ableit-)Elektroden in bipolare und unipolare Ableitungen einteilen.

Bei einer bipolaren Ableitung wird die elektrische Spannung zwischen zwei gleichberechtigten Punkten der Körperoberfläche registriert, zum Beispiel zwischen dem rechten Arm und dem linken Arm.

Die unipolare Ableitung hingegen misst die Spannung zwischen einer differenten Elektrode und einem elektrischen "Nullpunkt", der als indifferente Elektrode oder Bezugselektrode bezeichnet wird. Um die indifferente Elektrode zu erhalten, werden jeweils zwei oder mehr Extremitätenelektroden über Widerstände zusammen geschaltet.

Ferner kann man die Ableitungen danach einteilen, zwischen welchen Positionen sie bestimmt werden. Dabei unterscheidet man bspw. die Extremitätenableitungen, die Potentialdifferenzen zwischen den Extremitäten messen, von den Brustwandableitungen, die durch Elektroden am Thorax bestimmt werden.

Ein konventionelles 12-Kanal-EKG registriert zum Beispiel parallel die Extremitätenableitungen nach ihren Entwicklern "Einthoven" und "Goldberger", sowie die Brustwandableitung nach "Wilson". Wobei Einthoven und Goldberger drei Kanäle und Wilson sechs Kanäle benötigt.

Daneben gibt es bspw. Spezialableitungen nach "Nehb", "Frank" und "Dower".

Die Ableitung nach Nehb gilt als Ergänzungsableitung, die bei speziellen klinischen Fragestellungen zum Einsatz kommt. Das gilt auch für die im Rahmen der Vektorkardiografie eingesetzte Ableitung nach Frank.

Das von Frank beschriebene Ableitsystem basiert auf einer Vektorbetrachtung eines EKG, wobei ein Momentanvektor des EKG in die X, Y und Z- Komponenten eines orthogonalen Koordinatensystems zerlegt wird. Nach Frank sind lineare Gleichungen bekannt, mit denen aus den Potentialdifferenzen der Elektroden eine Transformation in die drei Ableitungen X, Y und Z möglich ist. Die gewonnenen drei Ableitungen beinhalten alle Informationen des EKG und ermöglichen eine vektorielle Darstellung der elektrischen Aktivität in verschiedenen Ebenen, bspw. durch zweidimensionale Darstellung jeweils zweier Ableitungen als so genannte "Vektorschleifen". Ferner sind Gleichungen bekannt, mit Hilfe derer die herkömmliche 12-Kanalableitung aus den drei orthogonalen Ableitungen nach Frank möglich ist.

Mit dem Ableitungssystem nach Dower ist es möglich, über fünf Elektroden (E, A, S und I) einschließlich einer zusätzlichen Erdelektrode, die orthogonalen Ableitungen X, Y und Z zu berechnen. Dower leitet drei Ableitungen von vier Elektroden ab, wobei eine Elektrode als gemeinsamer Bezugspol genutzt wird. Analog zum Prozedere der Frank-Transformation, kann auch aus dem Ableitsystem nach Dower, das herkömmliche 12 Kanal-EKG berechnet werden.

Bei manchen Ausführungsbeispielen wird eine Ableitung nach Einthoven, Goldberger, Wilson, Nehb, Frank oder Dower bei der Aufnahme des Elektrokardiogramms verwendet. Eine Elektrokardiogrammvorrichtung zum Aufnehmen eines Elektrokardiogramm weist dementsprechend je nach Ausführungsbeispiel die für das jeweilige Ableitsystem benötigte Anzahl von Elektroden auf (bspw., 2, 4, 5, 6, 8 oder 10 Elektroden), wobei die Anzahl der Elektroden beliebig ist und sich nach dem verwendeten, ebenfalls beliebigen Ableitsystem richtet.

Die Permutation bzw. Anordnung der Elektroden und die daraus resultierende Abweichung eines gemessenen EKGs von einem vorherigen wird durch ein Verfahren zum Aufnehmen eines Elektrokardiogramms ermittelt, das bspw. in einer Steuerung einer Elektrokardiogrammvorrichtung, bspw. einem Elektrokardiographen, abläuft. Dazu weist die Elektrokardiogrammvorrichtung bspw. neben der Steuerung wenigstens zwei Elektroden zum Aufnehmen von Elektrokardiogrammsignalen und ein Speichermittel auf, in dem wenigstens ein Referenz-Elektrokardiogramm gespeichert ist.

Zunächst messen wenigstens zwei Elektroden, die bspw. in einer vorgegeben Position an einem Menschen angebracht sind, Potentialunterschiede an der Hautoberfläche und senden entsprechende Signale über Signalleitungen, bspw. an eine Steuerung. Je nach Ausführungsbeispiel und je nach Ableitung kommt eine unterschiedliche Anzahl von Elektroden zur Anwendung, wie bspw. auch vier, fünf, sechs oder zehn Elektroden.

Kommen mehrere Elektroden zum Einsatz, so misst die Steuerung die Potentialunterschiede normalerweise immer zwischen denselben Elektroden, die allerdings - wie oben beschrieben - aufgrund fehlerhafter Positionierung bei aufeinanderfolgenden Messungen unterschiedlich angeordnet sein können oder es können die Signalleitungen bspw. beim Einstecken in die entsprechenden Eingänge des Elektrokardiographen vertausch sein. Eine solche Vertauschung "merkt" der Elektrokardiograph bzw. seine Steuerung nicht, sondern er geht bei der Messung immer davon aus, dass die Elektroden wie vorgegeben angeordnet sind.

Die Steuerung berechnet aus den empfangenen Elektrokardiogrammsignalen ein aktuelles Elektrokardiogramm, wobei sie dabei eine Anordnung oder Permutation der Elektroden, wie sie bspw. vorgegeben ist, annimmt.

Im nächsten Schritt ermittelt die Steuerung eine Abweichung des berechneten aktuellen Elektrokardiogramms von einem Referenz-Elektrokardiogramm, bspw. nach dem Gaußschen Fehlerverfahren. Das Referenz-Elektrokardiogramm hat bspw. die Steuerung zuvor in einem Speicher abgelegt und stammt normalerweise von demselben Subjekt, von dem die aktuellen Elektrodensignale stammen. In manchen Ausführungsbeispielen ist das Referenz-Elektrokardiogramm mit einer Standard-Konfiguration der Elektroden aufgenommen worden, während es bei anderen mit einer nicht standardisierten oder permutierten Anordnung aufgenommen worden ist.

Die ermittelte Abweichung kann die Steuerung bspw. in einem Speicher ablegen. Als Nächstes nimmt die Steuerung eine weitere Anordnung der Elektroden an und berechnet auf Grundlage dieser Permutation wiederum ein aktuelles Elektrokardiogramm, basierend auf denselben empfangenen Elektrokardiogrammsignalen. Auch für dieses erneut berechnete zweite aktuelle Elektrokardiogramm, ermittelt die Steuerung eine (zweite) Abweichung von dem Referenz-Elektrokardiogramm.

Diesen Ablauf wiederholt die Steuerung bis für jede mögliche Permutation für die Elektrodenanordnung ein aktuelles Elektrokardiogramm berechnet und eine zugehörige Abweichung vom Referenz-Elektrokardiogramm ermittelt ist.

Die Anzahl der Permutationen hängt von der Anzahl der verwendeten Elektroden ab. Bei zwei Elektroden gibt es zwei mögliche Anordnungen, bei vier Elektroden hingegen Fakultät(4), das heißt 24 Permutationen. Allgemein ist die Zahl der Permutationen durch die Funktion gegeben: Fakultät(Anzahl Elektroden).

Nachdem ein Teil oder-je nach Ausführungsbeispiel - alle Permutationen berechnet wurden, ermittelt die Steuerung die Abweichung die minimal war. Zusätzlich kann sie das zugehörige berechnete aktuelle Elektrokardiogramm und damit letztlich auch die Anordnung der Elektroden ermitteln, für die die Abweichung zwischen dem Referenz-Elektrokardiogramm und dem aktuellen Elektrokardiogramm minimal ist, und ebenfalls ausgeben.

Bei manchen Ausführungsbeispielen gibt die Steuerung dieses Elektrokardiogramm, das zur minimalen Abweichung gehört, als das aktuelle Elektrokardiogramm aus. Bei manchen Ausführungsbeispielen wird nur die minimale Abweichung ausgeben oder dies zusammen mit dem zugehörigen Elektrokardiogramm. Bei wieder anderen Ausführungsbeispielen wird bspw. ein abgeleitetes Signal von der minimalen Abweichung und/oder von dem Elektrokardiogramm ausgegeben.

Durch Ermittlung des Elektrokardiogramms mit der minimalen Abweichung, d.h. letztlich durch die (indirekte) Ermittlung der tatsächlichen Elektrodenanordnung, können die jeweiligen Elektroden mit beliebigen Signaleingängen der Steuerung verbunden werden. Mit anderen Worten, auch bei einer Vertauschung der Elektroden, bspw. beim aktuellen und/oder beim Referenz-Elektrokardiogramm, kann trotzdem das "richtige" Elektrokardiogramm ausgegeben werden und eine Veränderung von physiologischen Parametern kann anhand des "richtigen" ermittelten EKGs und/oder anhand der ermittelten minimalen Abweichung ermittelt werden.

Die Ausgabe des Elektrokardiogramms erfolgt auf verschiedene Art und Weise. Zum Beispiel kann das Elektrokardiogramm auf einem Anzeigemittel, bspw. Bildschirm oder Plotter, ausgegeben werden. Andererseits kann das Elektrokardiogramm auch an einen Speicher ausgegeben werden, um bspw. dort abgelegt zu werden.

Das Elektrokardiogramm für eine angenommene Elektrodenanordnung berechnet die Steuerung bspw. durch Addition der Potentiale, die sie von den jeweiligen Elektroden empfangen hat. Dabei können immer Potentiale von jeweils zwei Elektroden addiert werden. Bei manchen Ausführungsbeispielen werden aber auch mehr als zwei Potentiale addiert. Beispielsweise können der Mittelwert von zwei Elektrodenpotentialen und der Wert einer dritten Elektrode addiert werden.

Bei manchen Ausführungsbeispielen ermittelt bspw. die Steuerung zusätzlich aus der minimalen Abweichung und/oder aus dem zugehörigen aktuellen Elektrokardiogramm, ein Veränderung eines physiologischen Parameters, wie bspw. Durchblutung, Pumpfunktion des Herzens, Herzrhythmusstörungen und dergleichen.

Erkennt bspw. die Steuerung eine Veränderung eines physiologischen Parameters, so gibt sie bei manchen Ausführungsbeispielen ein Warnsignal aus, sodass der Patient in der Lage ist zu beurteilen, ob er bspw. einen Arzt aufsuchen oder einen Notarzt rufen muss. Um dem Patienten die Beurteilung seines aktuellen Herzzustandes zu erleichtern, gibt die Steuerung bspw. in Abhängigkeit der Stärke der Abweichung einen Zahlenwert aus, der die Stärke der physiologischen Veränderung repräsentiert. Bei manchen Ausführungsbeispielen gibt die Steuerung das Alarmsignal bspw. aus, wenn die minimale Abweichung einen vorgegebenen Schwellwert übersteigt. Zusätzlich kann die Steuerung auch für verschiedene Schwellwerte unterschiedliche Alarmsignale ausgeben. Auf diese Weise kann die Steuerung dem Patienten das Alarmsignal bspw. in Form einer Ampel signalisieren. Bei einem ersten niedrigen Schwellwert, bekommt der Patient ein grünes Leuchtsignal zu sehen, was ihm signalisiert, dass keine auffällig EKG-Veränderung gemessen werden konnte. Liegt die minimale Abweichung über einem zweiten Schwellwert, aber unter einem dritten, so bekommt er bspw. ein gelbes Signal zu sehen, was ihm andeutet, dass er zwar noch nicht sofort einen Arzt aufsuchen muss, er sich allerdings demnächst in Behandlung geben sollte. Ein rotes Signal, welches oberhalb des dritten Schwellwertes erscheint, signalisiert dem Patienten, dass er bspw. umgehend einen Arzt aufsuchen oder gar den Notarzt verständigen sollte. Bei manchen Ausführungsbeispielen gibt die Steuerung auch direkt die Abweichung aus. Bei manchen Ausführungsbeispielen gibt die Steuerung zusätzlich noch die ermittelte Elektrodenanordnung aus, bei der die Abweichung minimal ist.

Zurückkommend zu Fig. 1 veranschaulicht diese, wie an einem Patienten 1 eine erste Anordnung von vier Elektroden 2, 3, 4, 5 an den jeweiligen Positionen 6, 7, 8, 9 angebracht ist, um ein Referenz-EKG zu messen. In dieser ersten Anordnung befindet sich die Elektrode 2 an der Position 6, die Elektrode 3 an der Position 7, die Elektrode 4 an der Position 8 und die Elektrode 5 an der Position 9.

Eine Messung der Elektrodensignale, die in der ersten Anordnung nach Fig. 1 angeordnet sind, ergibt bspw. das EKG, wie es in Fig. 2 dargestellt ist. Dieses EKG entsteht durch Messen des zeitlichen Verlaufes der Potentialdifferenzen zwischen jeweils der Null-Position, d.h. Position 6, und einer der verbleibenden drei Positionen 7, 8 und 9:

$$V(\text{Elektrode 3}, t) - V(\text{Elektrode 2}, t) = V_1(\text{Position 7, Position 6}, t);$$

$$V(\text{Elektrode 4}, t) - V(\text{Elektrode 2}, t) = V_2(\text{Position 8, Position 6}, t);$$

und

$$V(\text{Elektrode 5}, t) - V(\text{Elektrode 2}, t) = V_3(\text{Position 9, Position 6}, t);$$

wobei das "V" für die entsprechend gemessene Potentialspannung steht und "t" die Zeitvariable angibt. Der zeitliche Verlauf der jeweils gemessenen Potentialdifferenzen $V_1$, $V_2$, $V_3$ ist in Fig. 2 durch die Kurven 10, 11 bzw. 12 dargestellt, d.h. $V_1$ entspricht der dunklen Kurve 10, $V_2$ entspricht der mittelgrauen Kurve 11 und $V_3$ entspricht der hellen Kurve 12.

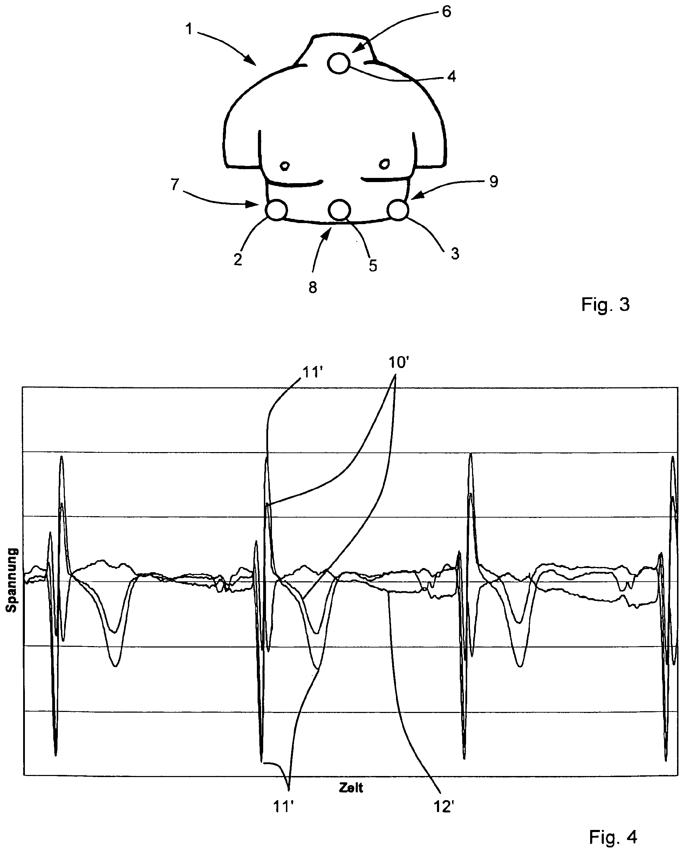

Eine Permutation der Elektroden, bspw. Elektrode 4 auf Position 6, Elektrode 2 auf Position 7, Elektrode 5 auf Position 8 und Elektrode 3 auf Position 9, wie in Fig. 3 veranschaulicht, führt zu einem anderen EKG als das in Fig. 4 gezeigte. Dieses unterscheidet sich von dem Referenz-EKG nach Fig. 2, wenn man die Potentiale zwischen den gleichen Elektroden misst wie bei dem Referenz-EKG, wie es ein Elektrokardiograph macht, da er nicht "weiß", dass die Elektroden vertauscht sind:

$$V(\text{Elektrode 3}, t) - V(\text{Elektrode 2}, t) = V_1(\text{Position 9, Position 7}, t);$$

$$V(\text{Elektrode 4}, t) - V(\text{Elektrode 2}, t) = V_2(\text{Position 6, Position 7}, t);$$

und

$$V(\text{Elektrode } 5, t) - V(\text{Elektrode } 2, t) = V_3(\text{Position } 8, \text{Position } 7, t).$$

Da der Elektrokardiograph immer zwischen den gleichen Elektroden/Signaleingängen die Potentialunterschiede misst, folgt entsprechend aus den Gleichungen, dass die Potentiale zwischen anderen Positionen bestimmt sind als beim Referenz-EKG. Folglich sind die resultierenden Kurven 10', 11' und 12' für die Potentiale $V_1$, $V_2$ und $V_3$ anders als diejenigen Kurven 10, 11 und 12 des Referenz-EKGs nach Fig. 2.

Durch eine entsprechende Umrechnung bzw. Permutation der Elektrodenanordnung, lassen sich die Potentiale zwischen den Positionen bestimmen bzw. berechnen, wie sie beim Referenz-EKG nach Fig. 2 verwendet wurden:

$$V_1(\text{Position } 7, \text{Position } 6, t) = - (V(\text{Elektrode } 4, t) - V(\text{Elektrode } 2, t));$$

$$V_2(\text{Position } 8, \text{Position } 6, t) = - (V(\text{Elektrode } 4, t) - V(\text{Elektrode } 2, t)) +$$
$$+ V(\text{Elektrode } 5, t) - V(\text{Elektrode } 2, t);$$

$$V_3(\text{Position } 9, \text{Position } 6, t) = - (V(\text{Elektrode } 4, t) - V(\text{Elektrode } 2, t)) +$$
$$+ V(\text{Elektrode } 3, t) - V(\text{Elektrode } 2, t);$$

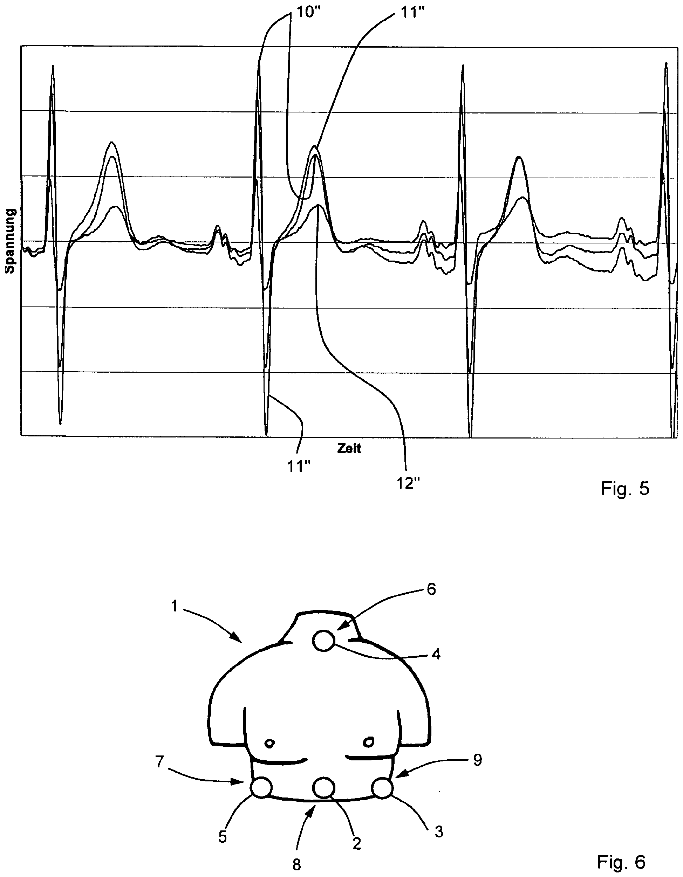

Diese Umrechung führt zu dem EKG nach Fig. 5, bei welchem $V_1$ der Kurve 10", $V_2$ der Kurve 11" und $V_3$ der Kurve 12" entspricht. Dieses umgerechnete EKG entspricht im Wesentlichen dem Referenz EKG nach Fig. 2, wie sich leicht aus einem Vergleich der beiden EKG aus den Fig. 2 und Fig. 5 erkennen lässt, da die tatsächliche Anordnung der Elektroden für die Berechnung angenommen wurde. Mit anderen Worten, diese Umrechnung bildet mathematisch die vertauschte Elektrodenanordnung und die zugehörigen Potentialverläufe auf die "richtige" des Referenz-EKGs ab.

Diese Umrechnung entspricht letztlich einer Vektoraddition. So ergibt bspw. die Addition des Vektors zwischen den Positionen 6 und 7 in Fig. 1 mit dem Vektor zwischen den Positionen 7 und 8 den Vektor $V_2$, der zwischen den Positionen 6 und 8 liegt. Analog ergibt die Addition der Vektoren zwischen den Positionen 6, 7 und 7, 9 den Vektor $V_3$, der zwischen den Positionen 9 und 6 liegt. Dieser Vektoraddition liegt die Annahme zugrunde, dass in einem Vektordreieck, z.B. zwischen den Positionen 6, 7, 8 oder 6, 7, 9 die Summe der Potentiale konstant ist.

Eine weitere Permutation der Elektrodenanordnung zeigt Fig. 6, bei welcher sich Elektrode 4 auf Position 6, Elektrode 5 auf Position 7, Elektrode 2 auf Position 8 und Elektrode 3 auf Position 9 befindet. Damit ergibt sich für die Potentialberechnung Folgendes, wenn man wiederum, wie der Elektrokardiograph, davon ausgeht, dass die Elektroden sich eigentlich an den Positionen nach Fig. 1 befinden:

$$V(\text{Elektrode } 3, t) - V(\text{Elektrode } 2, t) = V_1(\text{Position } 9, \text{Position } 8, t);$$

$$V(\text{Elektrode } 4, t) - V(\text{Elektrode } 2, t) = V_2(\text{Position } 6, \text{Position } 8, t);$$

und

$$V(\text{Elektrode } 5, t) - V(\text{Elektrode } 2, t) = V_3(\text{Position } 7, \text{Position } 8, t).$$

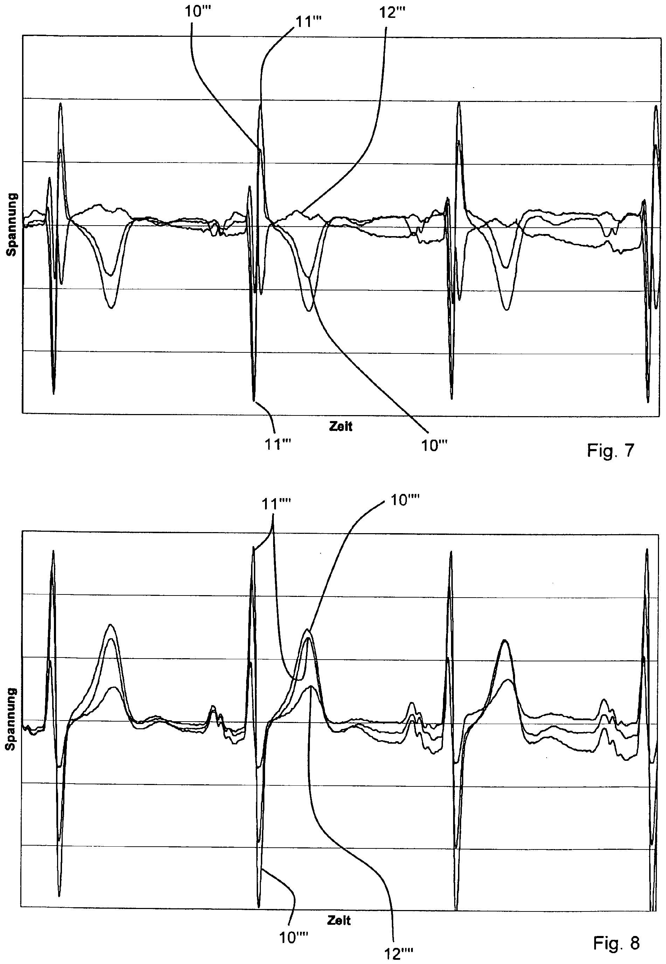

Das resultierende EKG zeigt Fig. 7, wobei die Kurve 10''' den Potentialverlauf $V_1$, Kurve 11''' den Potentialverlauf $V_2$ und Kurve 12''' den Potentialverlauf $V_3$ darstellt.

Nimmt man nun die gleiche Elektrodenanordnung an, wie im Zusammenhang Fig. 5 erörtert, so gelangt man zu dem folgenden EKG:

$$V_1(\text{Position } 7, \text{Position } 6, t) = - (V(\text{Elektrode } 4, t) - V(\text{Elektrode } 2, t));$$

$$V_2(\text{Position 8, Position 6, t}) = - (V(\text{Elektrode 4, t}) - V(\text{Elektrode 2, t})) +$$
$$+ V(\text{Elektrode 5, t}) - V(\text{Elektrode 2, t});$$

$$V_3(\text{Position 9, Position 6, t}) = - (V(\text{Elektrode 4, t}) - V(\text{Elektrode 2, t})) +$$
$$+ V(\text{Elektrode 3, t}) - V(\text{Elektrode 2, t});$$

Das aus dieser Berechnung resultierende EKG ist in Fig. 8 dargestellt und die Kurven 10'''', 11''''', 12'''' stellen entsprechend die Potentialverläufe $V_1$, $V_2$ bzw. $V_3$ dar. Wie leicht aus einem Vergleich des EKGs aus Fig. 8 mit dem Referenz-EKG nach Fig. 2 sichtbar ist, sind die Kurven 10'''' und 11'''' bezüglich dem Referenz-EKG vertauscht. Die angenommene Elektrodenanordnung war folglich nicht richtig - was nicht überrascht, denn der obigen Berechnung des EKGs nach Fig. 8 liegt die angenommene Permutation der Fig. 3 zugrunde, die sich sichtbar von derjenigen von Fig. 6 unterscheidet.

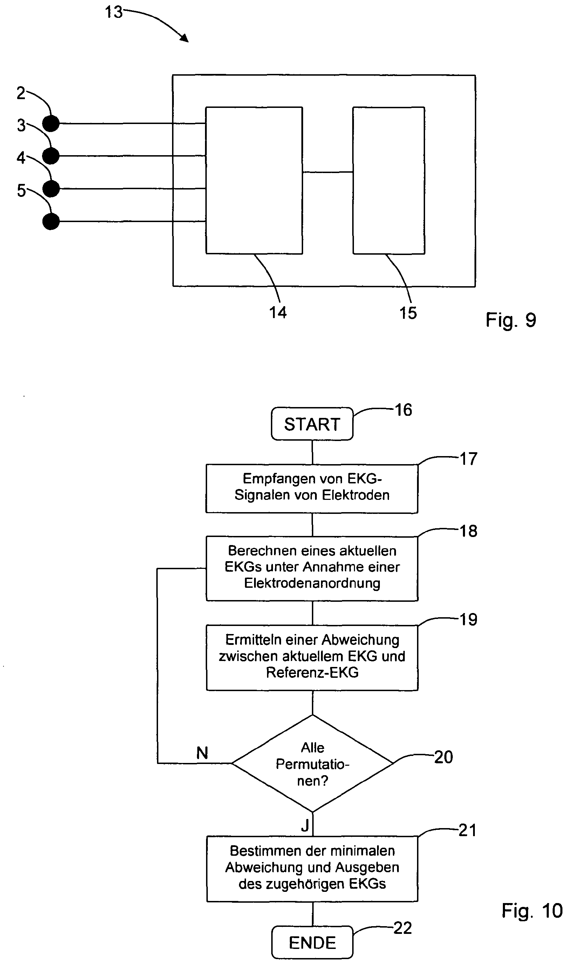

Die vorher gezeigten Elektrokardiogramme können bspw. mit einem mobilen EKG-Gerät 13, wie es schematisch in Fig. 9 dargestellt ist, aufgenommen werden. Das mobile EKG-Gerät umfasst neben den Elektroden 2, 3, 4, 5 eine Steuerung 14 und einen Speicher 15. Das EKG-Gerät bzw. die Steuerung des EKG-Geräts ist dazu ausgelegt, das Verfahren nach Fig. 10 auszuführen, das bei 16 beginnt und bei 22 endet.

Dazu empfängt die Steuerung 14 in einem ersten Schritt 17 von den an einem Patienten angelegten Elektroden 2, 3, 4, 5 entsprechende Elektroden-Signale und wertet diese aus. So empfängt das EKG-Gerät 13 zuerst EKG-Signale von den Elektroden 2, 3, 4, 5 in einer Anordnung, wie sie in Fig. 1 veranschaulicht ist. Aus diesen Elektrodensignalen berechnet die Steuerung die entsprechenden Potentialverlaufskurven 10, 11, 12, wie in Fig. 2 dargestellt. Dieses daraus resultierende EKG legt das EKG-Gerät 13 dann im Speicher 14 als Referenz-EKG für einen späteren Vergleich ab.

Wenn der Patient nun zu einem späteren Zeitpunkt wiederum die Elektroden 2, 3, 4, 5 an sich anlegt, so berechnet die Steuerung in einem Schritt 18 aus den nun aktuell empfangenen EKG-Signalen ein (erstes) aktuelles EKG, wobei die Steuerung 14 dabei eine erste Elektrodenanordnung annimmt - bspw. die Anordnung des Referenz-EKGs. Der Patient kann dabei die Elektroden gegenüber der Anordnung, die zum Referenz-EKG gehört, vertauscht haben. Oder der Patient hat bspw. eine Vertauschung beim Einstecken der Signalleitungen der Elektroden in das EKG-Gerät vorgenommen.

Die Steuerung 14 geht dann bei der Berechnung des aktuellen EKGs von der Elektrodenanordnung aus, mit der sie das Referenz-EKG aufgenommen hat. Allerdings ist es nicht notwendig, dass die Steuerung 14 die Elektrodenanordnung des Referenz-EKGs kennt, da die Steuerung alle möglichen Elektrodenanordnungen im Folgenden annimmt und die minimale Abweichung berechnet und so selbst bei unbekannter Anordnung für das Referenz-EKG das Elektrokardiogramm ermitteln kann, das dem Referenz-EKG entspricht. Die Berechnung des aktuellen EKGs mit der entsprechend angenommenen Elektrodenanordnung, erfolgt dann zum Beispiel analog wie oben beschrieben durch entsprechende Permutation der Elektroden und Potentialaddition.

In einem nächsten Schritt 19 ermittelt die Steuerung 14 eine Abweichung zwischen dem aktuell berechneten EKG, das auf der angenommenen Elektrodenanordnung basiert, und dem Referenz-EKG, das im Speicher 15 abgelegt ist. Die Permutation der Elektroden 2, 3, 4, 5 kann dabei bspw. so sein, wie in Fig. 3 oder Fig. 6 dargestellt ist.

In einem Schritt 20 überprüft die Steuerung 14, ob sie bereits alle möglichen Permutationen, das heißt Elektrodenanordnungen, durchgeprüft und mit dem Referenz-EKG verglichen hat. Falls dies nicht der Fall ist, springt die Steuerung 14 zum Schritt 18 zurück und ermittelt so lange, basierend auf angenommenen Elektrodenanordnungen, aktuelle Elektrokardiogramme bis die Bedingung 20 erfüllt ist. Die dabei jeweils ermittelten Abweichungen zwischen dem gerade aktuell ermittelten EKG unter Annahme einer entsprechenden Elektrodenanordnung legt die Steuerung 14 dabei ebenfalls im Speicher 15 ab. Wenn die Steuerung 14 alle Permutationen berechnet und verglichen hat und die zugehörigen Abweichungen bestimmt hat, so bestimmt sie im Schritt 21 die minimale Abweichung aus den ermittelten Abweichungen und gibt bspw. das zur minimalen Abweichung zugehörige EKG oder die minimale Abweichung selbst oder ein davon abgeleitetes Signal bspw. auf einer Anzeige (nicht gezeigt) des EKG-Geräts 13 aus.

Die Steuerung kann aus der ermittelten minimalen Abweichung auf eine Veränderung eines physiologischen Parameters schließen. Ein solcher Parameter kann bspw. die Pumpleistung, die Durchblutung des Herzens oder eine Herzrhythmusstörung betreffen. Dabei kann die Steuerung je nach Einstellung bspw. die Abweichung in Form eines Zahlenwertes ausgeben oder in Form einer farbigen Anzeige.

Bei manchen Ausführungsbeispielen umfasst das EKG-Gerät 13 drei Leuchtdioden, eine grüne, gelbe und eine rote. Die Steuerung vergleicht die ermittelte minimale Abweichung mit vorgegebenen Schwellwerten, die zu den entsprechenden Farben der LEDs gehören. Somit kann das EKG-Gerät 13 dem Patienten, wie bei einer Ampel, signalisieren, wie sein Zustand ist. Dabei bedeutet bspw. grün, dass keine signifikanten EKG-Veränderungen gemessen werden können, die eine Verschlechterung des Gesundheitszustandes darstellen könnten; gelb, dass er demnächst einen Arzt aufsuchen soll und rot, dass akute Gefahr besteht. Die Steuerung 14 verfährt dabei bspw. so, dass bei einer minimalen Abweichung, die unterhalb eines ersten vorgegebenen Schwellwertes liegt, die grüne LED leuchtet. Liegt die minimale Abweichung zwischen dem ersten und ein zweiten vorgegebenen Schwellwert, so leuchtet die gelbe LED auf. Liegt die minimale Abweichung über dem zweiten Schwellwert, so leuchtet die rote LED auf.

Die Ermittlung der minimalen Abweichung dient bei manchen Ausführungsbeispielen dazu, die tatsächliche Elektrodenanordnung zu bestimmen und damit das EKG, welches zur minimalen Abweichung gehört. Danach kann die Steuerung bei manchen Ausführungsbeispielen eine weitere Analyse und bspw. einen Detailvergleich zwischen dem aktuellen EKG und dem Referenz-EKG vornehmen und somit spezifische Abweichung, bspw. zwischen einander entsprechenden Potentialverläufen (bspw. zwischen 10 und 10", 11 und 11", 12 und 12") und Teilabschnitten davon vornehmen. Bei manchen Ausführungsbeispielen basiert dann die weitere Ausgabe eines spezifischen Alarmsignals bspw. auf einer derartigen spezifischen Analyse. So kann bspw. das EKG-Gerät je nach physiologischem Parameter ein spezifisches Alarmsignal ausgeben.

## Patentansprüche

1.  Verfahren zum Aufnehmen eines Elektrokardiogramms, umfassend die folgenden durch eine Steuerung ausgeführten Schritte:

    a) Berechnen und Speichern eines Referenz-Elektrokardiogramms aus gemessenen Referenz-Elektrokardiogrammsignaler;
    b) Empfangen von aktuellen Elektrokardiogrammsignalen von Elektroden (2, 3, 4, 5);
    c) Berechnen eines aktuellen Elektrokardiogramms aus den empfangenen aktuellen Elektrokardiogrammsignalen unter Annahme einer Anordnung der Elektroden (2, 3, 4, 5);
    d) Ermitteln einer Abweichung basierend auf einem Vergleich von Potentialverläufen des berechneten aktuellen Elektrokardiogramms mit Potentialverläufen des gespeicherten Referenz-Elektrokardiogramms;
    e) Wiederholen der Schritte c) und d) so lange, bis jede mögliche Elektrodenanordnung wenigstens einmal angenommen wurde, wobei bei jeder Wiederholung eine andere Elektrodenanordnung angenommen wird; und
    f) Bestimmen welche der ermittelten Abweichungen minimal ist.

2.  Verfahren nach Anspruch 1, bei welchem das aktuelle Elektrokardiogramm ausgegeben wird, bei welchem die Abweichung minimal ist.

3.  Verfahren nach Anspruch 1 oder 2, bei welchem das Referenz-Elektrokardiogramm an demselben Subjekt (1) aufgenommen wurde, wie die aktuellen Elektrokardiogrammsignale.

4.  Verfahren nach einem der vorhergehenden Ansprüche, bei welchem das aktuelle Elektrokardiogramm auf einer Addition der jeweiligen Potenziale der Elektrodensignale basiert.

5.  Verfahren nach einem der vorhergehenden Ansprüche, bei welchem die aktuellen Elektrokardiogrammsignale mit einer Ableitung nach Einthoven, Goldberger, Wilson, Nehb, Frank oder Dower empfangen werden.

6.  Verfahren nach Anspruch 1, bei welchem in Abhängigkeit der Stärke der Abweichung ein Zahlenwert ausgegeben wird.

7.  Verfahren nach einem der vorhergehenden Ansprüche, bei welchem die ermittelte minimale Abweichung ausgegeben wird.

8.  Elektrokardiogrammvorrichtung zum Aufnehmen eines Elektrokardiogramms, umfassend:

    - Elektroden (2, 3, 4, 5) zum Aufnehmen von Elektrokardiogrammsignalen;
    - wenigstens eine Steuerung (14), die eingerichtet ist, das Verfahren nach einem der Ansprüche 1 bis 7 auszuführen.
    - wenigstens ein Speichermittel (15) zum Speichern des Referenz-Elektrokardiogramms.

9.  Elektrokardiogrammvorrichtung nach Anspruch 8, welche ein Ableitsystem nach Einthoven, Goldberger, Wilson, Nehb, Frank oder Dower umfasst.

**10.** Elektrokardiogrammvorrichtung nach Anspruch 8 oder 9, welche eingerichtet ist, ein Alarmsignal auszugeben, wenn die ermittelte Abweichung, die als minimal bestimmt ist, einen vorgegebenen Schwellwert übersteigt.

**11.** Elektrokardiogrammvorrichtung nach Anspruch 10, welche eingerichtet ist, für verschiedene Schwellwerte verschiedene Alarmsignale auszugeben.

**Claims**

**1.** A method for recording an electrocardiogram, comprising the following steps executed by a controller:

a) calculating and storing a reference electrocardiogram from measured reference electrocardiogram signals;
b) receiving current electrocardiogram signals from electrodes (2, 3, 4, 5);
c) calculating a current electrocardiogram from the received current electrocardiogram signals, assuming an arrangement of the electrodes (2, 3, 4, 5);
d) determining a deviation based on a comparison of potential curves of the calculated current electrocardiogram with potential curves of the stored reference electrocardiogram;
e) repeating the steps c) and d), until each possible electrode arrangement has been assumed at least once, wherein a different electrode arrangement is assumed for each repetition; and
f) determining which of the determined deviations is minimal.

**2.** Method according to claim 1, wherein the current electrocardiogram with the minimal deviation is outputted.

**3.** Method according to claim 1 or 2, wherein the reference electrocardiogram is recorded on the same subject (1) as the current electrocardiogram signals.

**4.** Method according to one of the preceding claims, wherein the current electrocardiogram is based on an addition of the respective potentials of the electrode signals.

**5.** Method according to one of the preceding claims, wherein the current electrode signals are received with a lead according to Einthoven, Goldberger, Wilson, Nehb, Frank, or Dower.

**6.** Method according to claim 1, wherein a numeric value is outputted depending on the amount of the deviation.

**7.** Method according to one of the preceding claims, wherein the determined minimal deviation is outputted.

**8.** An electrocardiogram apparatus for recording an electrocardiogram, comprising:

- electrodes (2, 3, 4, 5) for recording electrocardiogram signals;
- at least one controller (14), configured to perform the method according to one of claims 1 to 7.
- at least one storage means (15) for storing the reference electrocardiogram.

**9.** Electrocardiogram apparatus according to claim 8 comprising a lead system according to Einthoven, Goldberger, Wilson, Nehb, Frank or Dower.

**10.** Electrocardiogram apparatus according to claim 8 or 9, which is configured to output an alarm signal when the deviation determined to be minimal exceeds a predefined threshold.

**11.** An electrocardiogram apparatus according to claim 10, which is configured to output different alarm signals for different thresholds.

**Revendications**

**1.** Procédé servant à enregistrer un électrocardiogramme, comprenant les étapes suivantes exécutées par une commande :

(a) le calcul et la mémorisation d'un électrocardiogramme de référence à partir de signaux d'électrocardiogramme de référence mesurés ;

b) la réception de signaux d'électrocardiogramme instantanés provenant d'électrodes (2, 3, 4, 5) ;

c) le calcul d'un électrocardiogramme instantané à partir des signaux d'électrocardiogramme instantanés reçus en prenant un agencement des électrodes (2, 3, 4, 5) ;

d) la détermination d'un écart sur la base d'une comparaison de variations de potentiel de l'électrocardiogramme instantané calculé et de variations de potentiel de l'électrocardiogramme de référence mémorisé ;

e) la répétition des étapes c) et d) jusqu'à ce que chaque agencement d'électrodes possible ait été pris au moins une fois, dans lequel pour chaque répétition un autre agencement d'électrodes est pris ; et

f) la détermination de celui des écarts déterminés, qui est minimal.

2. Procédé selon la revendication 1, dans lequel on fournit l'électrocardiogramme instantané, pour lequel l'écart est minimal.

3. Procédé selon la revendication 1 ou 2, où l'électrocardiogramme de référence a été enregistré au niveau du même sujet (1) que les signaux d'électrocardiogramme instantanés.

4. Procédé selon l'une quelconque des revendications précédentes, où l'électrocardiogramme instantané se base sur une addition des potentiels respectifs des signaux d'électrodes.

5. Procédé selon l'une quelconque des revendications précédentes, où les signaux d'électrocardiogramme instantanés sont reçus avec une dérivée selon Einthoven, Goldberger, Wilson, Nehb, Frank ou Dower.

6. Procédé selon la revendication 1, où l'on fournit une valeur numérique en fonction de l'ampleur de l'écart.

7. Procédé selon l'une quelconque des revendications précédentes, où l'on fournit l'écart minimal déterminé.

8. Dispositif d'électrocardiogramme servant à enregistrer un électrocardiogramme, comprenant :

- des électrodes (2, 3, 4, 5) servant à enregistrer des signaux d'électrocardiogramme ;
- au moins une commande (14), qui est mise au point pour exécuter le procédé selon l'une quelconque des revendications 1 à 7 ;
- au moins un moyen de mémorisation (15) servant à mémoriser l'électrocardiogramme de référence.

9. Dispositif d'électrocardiogramme selon la revendication 8, lequel comprend un système de dérivée selon Einthoven, Goldberger, Wilson, Nehb, Frank ou Dower.

10. Dispositif d'électrocardiogramme selon la revendication 8 ou 9, lequel est mis au point pour émettre un signal d'alarme quand l'écart déterminé, qui est défini comme étant minimal, dépasse une valeur de seuil prédéfinie.

11. Dispositif d'électrocardiogramme selon la revendication 10, lequel est mis au point pour émettre des signaux d'alarme différents pour différentes valeurs de seuil.

Fig. 1

Fig. 2

Fig. 3

Fig. 4

Fig. 5

Fig. 6

Fig. 7

Fig. 8

Fig. 9

START — 16

Empfangen von EKG-Signalen von Elektroden — 17

Berechnen eines aktuellen EKGs unter Annahme einer Elektrodenanordnung — 18

Ermitteln einer Abweichung zwischen aktuellem EKG und Referenz-EKG — 19

N — Alle Permutatio-nen? — 20

J

Bestimmen der minimalen Abweichung und Ausgeben des zugehörigen EKGs — 21

ENDE — 22

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10065578 A1
- US 4850370 A
- US 20070232946 A1

**In der Beschreibung aufgeführte Nicht-Patentliteratur**

- **G. BALÁZS et al.** Intelligent Cardiac Telemonitoring System. *Computers in Cardiology,* 2004, vol. 31, 745-748
- **J. A. KORS.** Accurate Automatic Detection of Electrode Interchange in the Electrocardiogram. *American Journal of Cardiology,* 15. August 2001, vol. 88 (4), 396-399